# EUROPEAN PATENT SPECIFICATION

(11) **EP 2 995 374 B1**
(45) Date of publication and mention of the grant of the patent: **23.09.2020**
(21) Application number: 14184322.7
(22) Date of filing: 10.09.2014
(51) Int. Cl.: B01J 23/745, B01J 23/89, B01J 35/00, B01J 35/06, B01J 23/755, B01J 37/16, B01J 37/02

(54) **COMPOSITE MATERIAL CONSISTING OF A CELLULOSE FIBROUS HOST MATERIAL AND ZEROVALENT IRON NANOPARTICLES AND ITS PREPARATION AND USE AS CATALYST**
KOMPOSITMATERIAL BESTEHEND AUS EINEM TRÄGER AUS CELLULOSEFASERN UND ZERO-VALENTEN EISEN NANOPARTIKELN, HERSTELLUNG UND VERWENDUNG ALS KATALYSATOR
MATERIAUX COMPOSITE COMPOSEE D'UN MATERIAUX COMPOSEE DES FIBRES CELLULOSE ET DES NANOPARTICULE DE FER ZERO-VALENT ET SON PROCEDE DE PREPARATION ET UTILISATION COMME CATALYSEUR

(43) Date of publication of application: 16.03.2016
(73) Proprietor: Regional Centre of Advanced Technologies and Materials, Palacky University in Olomouc, 78371 Olomouc (CZ)
(72) Inventor: Kasibhatta, Kumara Ramanatha Datta, 560054 Bangalore, Karnataka (IN); Petala, Eleni, 68200 Evros (GR); Kasibhatta, Josena Datta, 560054 Bangalore, Karnataka (IN); Perman, Jason Alexander, Lutz, FL 33549 (US); Filip, Jan, 78371 Olomouc (CZ); Zboril, Radek, 77900 Olomouc (CZ)
(74) Representative: Hartvichova, Katerina

(56) References cited:
- EP-A1- 0 233 776
- WO-A2-2014/033642
- CN-A- 104 014 812
- US-A1- 2005 245 658
- US-A1- 2012 064 332
- NESRIN HORZUM ET AL: "Chitosan fiber-supported zero-valent iron nanoparticles as a novel sorbent for sequestration of inorganic arsenic", RSC ADVANCES, vol. 3, no. 21, 1 January 2013 (2013-01-01), page 7828, XP055170460, ISSN: 2046-2069, DOI: 10.1039/c3ra23454a
- SHILI XIAO ET AL: "Immobilization of Zerovalent Iron Nanoparticles into Electrospun Polymer Nanofibers: Synthesis, Characterization, and Potential Environmental Applications", JOURNAL OF PHYSICAL CHEMISTRY C, vol. 113, no. 42, 22 October 2009 (2009-10-22), pages 18062-18068, XP055170462, ISSN: 1932-7447, DOI: 10.1021/jp905542g
- WU L ET AL: "Preparation of cellulose acetate supported zero-valent iron nanoparticlesfor the dechlorination of trichloroethylene in water", JOURNAL OF NANOPARTICLE RESEARCH, KLUWER ACADEMIC PUBLISHERS, DORDRECHT, NL, vol. 7, no. 4-5, 1 October 2005 (2005-10-01), pages 469-476, XP009510672, ISSN: 1388-0764, DOI: 10.1007/S11051-005-4271-5

## Description

### Field of Art

The present invention relates to novel composite material useful as reactive magnetic filters containing zerovalent iron, the preparation and use thereof.

### Background Art

Nanoscale zerovalent iron (NZVI) particles is highly promising reactive material used for the removal of a wide range of chemicals like arsenic, halogenated hydrocarbons, organic compounds, anions, heavy metals, radionuclides and microbial contaminants from contaminated waters (Kharisov, B. I., H. V. R. Dias, et al. (2012). "Iron-containing nanomaterials: synthesis, properties, and environmental applications." Rsc Advances 2(25): 9325-9358.; Noubactep, C., S. Care, et al. (2012). "Nanoscale Metallic Iron for Environmental Remediation: Prospects and Limitations." Water Air and Soil Pollution 223(3): 1363-1382.).

Despite being extremely effective against several harmful contaminants, NZVI exhibits several physical weaknesses that limit the spectrum of its application in a broader range of bioremediation scenarios mainly in treatment of waste and drinking water. The known limitations are intimately connected to its high tendency towards aggregation, rapid sedimentation and spontaneous oxidation. These properties lead to a decrease in activity of NZVI after exposure to the pollutants, a factor that can restrict its extensive use in industrial scale.

In order to overcome the drawbacks of NZVI, in particular its tendency to agglomeration, several host materials such as polymers (Ponder, S. M., J. G. Darab, et al. (2000). "Remediation of Cr(VI) and Pb(II) aqueous solutions using supported, nanoscale zero-valent iron." Environmental Science & Technology 34(12): 2564-2569; Huang, Q., X. Shi, et al. (2008). "Tunable Synthesis and Immobilization of Zero-Valent Iron Nanoparticles for Environmental Applications." Environmental Science & Technology 42(23): 8884-8889), porous solids (Hoch, L. B., E. J. Mack, et al. (2008). "Carbothermal Synthesis of Carbon-supported Nanoscale Zero-valent Iron Particles for the Remediation of Hexavalent Chromium." Environmental Science & Technology 42(7): 2600-2605; Petala, E., K. Dimos, et al. (2013). "Nanoscale zero-valent iron supported on mesoporous silica: Characterization and reactivity for Cr(VI) removal from aqueous solution." Journal of Hazardous Materials 261(0): 295-306), functionalized clays (Hwang, Y., Y. C. Lee, et al. (2014). "Nanoscale zero-valent iron (nZVI) synthesis in a Mg-aminoclay solution exhibits increased stability and reactivity for reductive decontamination." Applied Catalysis B-Environmental 147: 748-755.), graphene, were tested so far (Jabeen, H., V. Chandra, et al. (2011). "Enhanced Cr(VI) removal using iron nanoparticle decorated graphene." Nanoscale 3(9): 3583-3585). These host materials are often costly, and the anchoring of NZVI on these supports may require complex procedures and may have a low reproducibility (Ali, I. (2012). "New Generation Adsorbents for Water Treatment." Chemical Reviews 112(10): 5073-5091).

Organic-fiber materials are cost effective, usually biodegradable, flexible, ecofriendly and oxygen rich (Samir, M. A. S. A., F. Alloin, et al. (2005). "Review of recent research into cellulosic whiskers, their properties and their application in nanocomposite field." Biomacromolecules 6(2): 612-626). They are in general formed by a porous scaffold composed of α-cellulose or other types of fibers.

### Disclosure of the Invention

The present invention relates to a composite material containing nanoscale zerovalent iron particles (NZVI) which consists of a cellulose fibrous host material and nanoscale zerovalent iron particles.

The cellulose fibrous host material is a cotton-fiber cloth, a linen-fiber cloth, or a filter paper, in particular ash-free filter paper.

The NZVIs are zerovalent iron (i.e., Fe⁰) particles having the dimensions (e.g., diameter for approximately spherical particles) of about 5 to about 500 nm, preferably about 100 nm. The resulting material is ferromagnetic, i.e., responsive to an external magnet, which facilitates its removal from treated mixtures when used for removal of pollutants.

The present invention further provides a method of preparation of the material containing nanoscale zerovalent iron particles, wherein cellulose fibrous material is impregnated with an alcoholic or aqueous iron (i.e., ferric or ferrous) salt solution, and subsequently the impregnated cellulose fibrous material is subjected to reduction to convert *in situ* the metal ions, i.e. the ferric/ferrous ions to nanoscale zerovalent iron particles.

The iron salt can be any iron salt of inorganic acid, or iron salt of organic acid, wherein said salt is soluble in water and/or alcohol. Preferably, the iron salt is ferric halogenide. More preferably, the iron salt is ferric chloride or ferric bromide, most preferably it is ferric chloride hexahydrate or ferric bromide hexahydrate. The term "iron salt" includes also hydrates and other suitable solvates of said salts.

The alcohol is C1-C4 alcohol or their mixture, more preferably methanol or ethanol.

The impregnation may be carried out by immersing the cellulose fibrous material in the metal salt solution, or by spraying the metal salt solution over the cellulose fibrous material, or by applying the metal salt solution using a suitable tool, e.g., a brush.

The reduction is carried out using a reduction agent such as sodium borohydride (NaBH₄). The successful coating of the cellulose fibrous material with ferric/ferrous cations and their reduction process to NZVIs can be easily monitored by bare eyes, since the impregnation of filter paper with the iron salt leads to formation of a bright-yellow material, which upon reduction turns black and it is then responsible to an external magnet.

More particularly, the present invention thus relates to a composite material containing nanoscale zerovalent iron particles which consists of a cellulose fibrous host material and nanoscale zerovalent iron particles and which is obtainable by the above-described process.

There is no need for any fibrous material pre-treatment. The preparation method is straightforward and does not rely on any costly starting materials or complex procedures. Any desired shape of the resulting material or any desired pattern of NZVIs in the resulting materials can be very easily achieved by cutting suitable shapes from the starting fibrous material or from the resulting composite material, or by applying the iron salt to the fibrous material with the relevant pattern. The present invention thus allows selective patterning of NZVI over large areas on FP which could be useful in systems with unusual shapes to optimize the available surface area.

It was verified that in the thus prepared composite material, the NZVI remained highly stable in air for at least one month and no special care has to be taken while storing or handling the composite material at room temperature or lower. There are no iron oxide/hydroxide phases present in the prepared material in quantity detectable by common instrumental techniques (i.e., < 10 wt.%). The loading of NZVI is easily controllable by adjusting the initial concentration of the iron salt used for the impregnation. It appears that the predominant phase of zerovalent iron present in the composite material is body-centered cubic alpha-Fe phase.

The composite material of the present invention may be used as a filter for wastewater treatment, in particular for remediation of heavy metals; or as a catalyst, in particular as a catalyst in hydroxylation reactions. The present invention allows the usage of reactive magnetic filters for the remediation of metals such as Cr(VI) or arsenic, or metal oxoanions such as arsenic oxoanion (AsO₄³⁻), and organic compounds such as phenols via simple filtration.

In order to be an efficient material to remove heavy metals such as Cr(VI) and/or other inorganic/organic pollutants in current water-treatment technologies, the material should display more than one of the following properties: i) high removal/remediation per material unit; ii) quick removal per time; iii) little disturbance to the host system; iv) recyclability; and v) easy adaptation to current and future host systems. For example, the state-of-art technologies such as NZVI on polymers fulfills items i and ii; NZVI on zeolites fulfills i, ii, iv; NZVI on clays fulfills items i, ii, listed above. The material of the present invention displays all five required properties.

### Brief description of Drawings

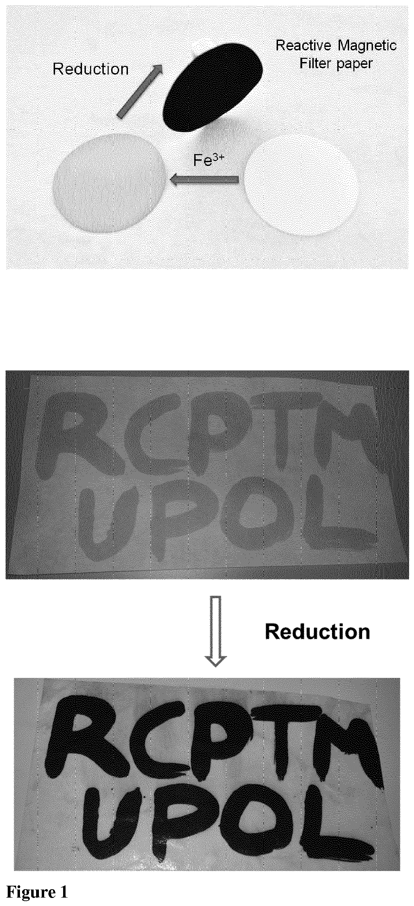

**Figure 1****.** Optical image showing different stages involved in the formation of reactive magnetic filter paper.
   Top: white circle - Whatmann, Grade 40, r = 55 mm filter paper - changes to yellow colored filter paper on is coating with FeCl₃•6H₂O solution which on chemical reduction turns to black colored magnetic paper.
   Bottom: Patterned filter paper, coating with FeCl₃•6H₂O solution (top) with paint brush, which on chemical reduction with NaBH₄ (followed by washing with water/ethanol) turns to black colored patterned paper (bottom).
**Figure 2****.** Transmission electron microscope (TEM) images of NZVI particle (black spherical particles) sizes in the range of 10-50 nm that are encapsulated along the cellulose fibers.
**Figure 3****.** X-ray powder diffraction pattern of filter paper (FP) and NZVI-modified filter paper (NZVI@FP), showing peaks at 2θ angle of 52.5 and 77.3o corresponding to the presence of (110) and (200) reflections of body-centered cubic (bcc) α-Fe.
**Figure 4****.** (a) Zero-field room-temperature and (b) in-field low-temperature (5 K/5 T) ⁵⁷Fe Mössbauer spectrum of the NZVI-modified filter paper (NZVI@FP) sample.
**Figure 5****.** (a) Hysteresis loops of the NZVI-modified filter paper (NZVI@FP) sample, measured at a temperature of 5 and 300 K and (b) ZFC/FC magnetization curves of the NZVI-modified filter paper (NZVI@FP) sample, measured under an external magnetic field of 0.01 T.
**Figure 6****.** Scheme showing the filtration setup (left). A stock solution of Cr(VI) (6.5 mg/L, 30 mL) was gravity feed through the filter paper (FP), filter paper with anchored *ex-situ* prepared NZVI particles (FP+NZVI) and NZVI-modified filter paper (NZVI@FP); the filtrate was further characterized for the unreacted Cr(VI). Plot showing (right) Cr (VI) removal efficiency for pure FP, ex-situ synthesized NZVI over FP and NZVI@FP.
**Figure 7****.** Formation of aromatic compounds as obtained by GC-MS analysis in the phenol hydroxylation process using NZVI-modified filter paper (NZVI@FP) (5 % wt) as catalytic membrane.
**Figure 8****.** Plot showing As(V) removal efficiency for NZVI-modified filter paper (NZVI@FP) (5 % wt).
**Figure 9****.** Top: Macroscopic optical changes of the cotton cloth before (left) and after (right) the two-step modification by NZVI particles; Bottom: The detailed scanning-electron microscopy (SEM) characterization of the NZVI-modified cotton fibres showing NZVI particles with a mean size of 300 nm.
**Figure 10****.** UV-Vis absorption spectroscopy showing concentration changes of Cr(VI) based on the slow pouring of the Cr(VI) solution (6.5 mg/L, 30 mL) through NZVI-containing cotton cloth (left) or NZVI-containing linen cloth (right).
**Figure 11****.** Top: Macroscopic optical changes of the linen cloth before (left) and after (right) the two-step modification by NZVI particles; Bottom: The detailed scanning-electron microscopy (SEM) characterization of the NZVI-modified linen fibres showing NZVI particles with a mean size of 250 nm.

### Examples

### Materials and methods

### Chemicals

All the chemical reagents were used without any further purification. Following chemicals were used in examples: Iron (III) chloride hexahydrate (FeCl₃.6H₂O), potassium dichromate (K₂Cr₂O₇), palladium chloride (K₂PdCl₆) 1,5-diphenylcarbazide, hydrogen peroxide (H₂O₂) from Sigma-Aldrich, ethanol, acetone analytical grade, sodium borohydride (NaBH4), concentrated phosphoric acid and Phenol from Merck. Filter paper (Grade 40, r = 55 mm) (FP) were obtained from Whatman; various cotton and linen cloths were obtained from local companies. Trifluoroacetic acid and hexamethyldisilazane as well as standards of phenol, hydroquinone and resorcinol were purchased from Sigma-Aldrich.

### Determination of hexavalent chromium and arsenic

UV-Vis spectroscopy was used for the determination of aqueous hexavalent chromium Cr(VI) by the 1,5-diphenylcarbazide method. Dissolved Cr(VI) reacts with diphenylcarbazide in acidic solution to form a red-purple "chromium 1,5-diphenylcarbohydrazide complex" that shows absorbance maximum at 542 nm. Diphenylcarbazide solution (10 mM) was prepared by dissolving 0.025 g 1,5-diphenylcarbohydrazide in 10 mL acetone. The solution was stored in a closed flask at 4 °C up to 7 days. Phosphoric acid solution was prepared by diluting 0.5 mL concentrated phosphoric acid in 10 mL deionized water. To each tested solution (3 mL) 120 µL of the diphenylcarbazide solution and 60 µL of the phosphoric acid solution were added. The solution was stirred and left for 10 minutes to allow complexation between Cr(VI) and diphenylcarbazide, resulting in a noticeable color change from colorless to violet. The calibration curves were initially determined by using the absorbance spectra of standard chromium solutions. Determinations of arsenic concentrations were carried out by atomic absorption spectroscopy (AAS) method.

### Remediation of Cr(VI) and As(V) through filtration

Composite material containing nanoscale zerovalent iron (NZVI) particles which comprises a fibrous host material and nanoscale zerovalent iron particles (5 wt% of NZVI) was used as filtration membrane for the remediation of Cr(VI) and As(V). 30 mL of Cr(VI) solution ([Cr(VI)]0 = 6.5 mg/L) was slowly added through these membranes and the filtrate was collected. For efficient remediation of Cr(VI), the filtrate is again passed through the same membrane. This procedure is repeated for 3 times. The filtrate obtained after 3 cycles was separated and tested by UV-Vis absorption spectroscopy to determine unreduced Cr(VI) by the 1,5-diphenylcarbazide method. 200 mL of As(V) solution (As(V) = 1 mg/L) was slowly added through these membranes and the filtrate was collected. For efficient remediation of As(V), the filtrate is again passed through the same membrane. This procedure is repeated for 5 times. The filtrate obtained after each cycle was separated and tested by AAS to determine arsenic concentration.

### Procedure and assessment of the phenol hydroxylation reaction

The reactions were performed in a conical flask under simultaneous stirring using a hotplate stirrer (Heidolph 3001K). 165 mg of phenol was dissolved in 17 mL deionized water. To this solution, 0.36 mL of H₂O₂ (30 %) was added (H₂O₂ / phenol molar ratio equal to 2:1). This reaction mixture is referred as initial solution and was stirred for 2 minutes at 40 °C and was allowed to pass through composite material containing nanoscale zerovalent iron (NZVI) particles which comprises a fibrous host material and nanoscale zerovalent iron particles (5 wt% of NZVI) supported on Coors™ Buchner funnel. The filtrate was collected and is referred as first filtration. For efficient hydroxylation the filtrate is again passed through the same membrane three times. This procedure was also tested for pure membrane (filter paper). In all these cases aliquots of 3 mL filtrate was taken immediately using a syringe filter (PTFE, 25 mm, 0.22 µm) and was centrifuged to obtain a clear solution, which was analyzed by GC and GC-MS.

### Characterization techniques

The composite material containing nanoscale zerovalent iron (NZVI) particles which comprises a fibrous host material and nanoscale zerovalent iron particles (5 wt% of NZVI) was sonicated for 2 minutes in ethanol. The obtained dispersion was drop casted on a carbon-coated copper grid. TEM images were taken employing a JEOL 2010F microscope operated at 200 kV (LaB6 cathode, resolution 0.19 nm) using carbon coated copper grid. Scanning electron microscopy (SEM) images were captured on a Hitachi 6600 FEG microscope operating in the secondary electron mode and using an accelerating voltage of 5 kV.

X-ray diffraction (XRD) patterns were recorded on an X'Pert PRO MPD diffractometer (PANalytical, Netherlands) using iron-filtered Co*K*_{α} radiation (*λ* = 0.178901 nm, 40 kV, 30 mA).

Zero-field ⁵⁷Fe Mössbauer spectrum of the studied sample was recorded at 300 K employing a Mössbauer spectrometer operating at a constant acceleration mode and equipped with a 50 mCi ⁵⁷Co(Rh) source. The in-field ⁵⁷Fe Mössbauer spectrum was measured at 5 K by placing the sample into a cryomagnetic system (Oxford Instruments) and exposed to an external magnetic field of 5 T, applied parallel to the direction of γ-rays propagation. The values of the isomer shift are reported with respect to α-Fe.

A superconducting quantum interference device (SQUID, MPMS XL-7, Quantum Design) has been used for the magnetic measurements. The hysteresis loops were collected at a temperature of 5 K and 300 K in external magnetic fields from - 5 T to + 5 T. The zero-field-cooled (ZFC) and field-cooled (FC) magnetization curves were recorded on warming in the temperature range from 5 to 300 K and in an external magnetic field of 0.01 T after cooling in a zero magnetic field and a field of 0.01 T, respectively.

The phenol conversion was monitored by a gas chromatograph (GC) Agilent 6820 equipped with capillary column Agilent DB-5 under the operation parameters: Inlet temperature: 300 °C, FID temperature: 300 °C, temperature ramp of the oven: 80 °C to 250 °C at a rate of 10 °C/min.

Hydroxylated products were identified by gas chromatography-mass spectrometry (GC-MS). The presence of hydroxyl group(s) was proved by chemical conversion to respective trimethylsilyl ethers performed as follows: 50 µL of aqueous sample solution was acidified with 20 µL of trifluoroacetic acid and derivatized-extracted with 500 µL of hexamethyldisilazane (HMDS) for 10 min. HMDS (upper) phase was analyzed by GC-MS using gas chromatograph Agilent 7890 A and mass selective detector Agilent 5976 C equipped with capillary column HP-5ms, 30 m x 0.25 mm x 0.25 µm under the operation parameters: Inlet temperature 280 °C, injection volume 1 µL, injection pulse 0.2 min at 20 Psi, temperature program 50 °C - 2 min - 10 °C/min - 300 °C - 15 min, electron ionization (70 eV), mass scan 29-520 m/z. Mass spectral library NIST 08 as well as mass spectra and retention times obtained from analysis of standards under identical conditions were used for reliable identification.

### Example 1

The preparation of NZVI embedded in filtration paper (FP) consisted of two simple steps, first one involving the impregnation of FP with ferric chloride salt followed by the chemical reduction with NaBH₄. In particular, 0.25 g of ferric chloride hexahydrate was dissolved in 2 mL ethanol. Ash free FP (Whatmann, Grade 40, *r* = 55 mm) was impregnated with the metallic solution followed by air-drying, procedure that gave a yellow colored FP (Fig. 1a). The reducing agent was prepared by dissolving 0.5 g of NaBH₄ in 100 mL of deionized water. The Fe³⁺ ions-coated FP was simply dipped in the NaBH₄ solution for 45 min, giving rise to a black colored magnetic paper (Figure 1). The composite was washed thoroughly with deionized water, then with absolute ethanol and finally dried under vacuum. The amount of NZVI in the FP was found to be -12 mg (i.e., 5% of the total weight of the composite). Similarly, various loadings of NZVI over FP were prepared by using appropriate amounts of FeCl₃·6H₂O and NaBH₄ with following proportions, e.g., 0.125 g of ferric chloride hexahydrate and 0.5 g of NaBH₄ gave -2.5% NZVI loading, 0.2 g of ferric chloride hexahydrate and 0.5 g of NaBH₄ gave -4% NZVI loading.

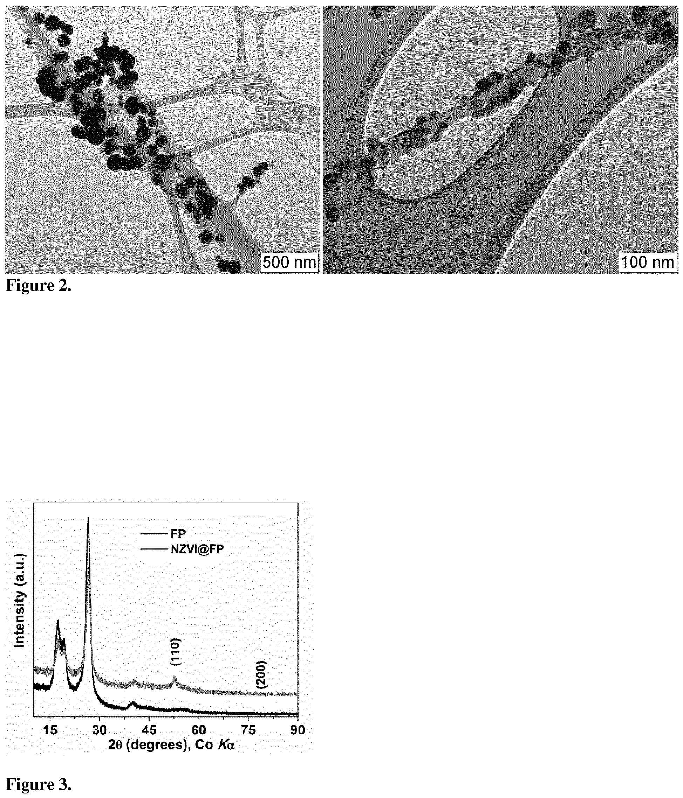

The nano-sized nature of the trapped NZVI particles, with dimensions varying in the range of 10-100 nm, was further validated by transmission electron microscopy (TEM) that revealed the entrapment of NZVI by the cellulose fibers (Figure 2). The chemical nature, crystallinity and magnetic characteristics of composite were evaluated with the aid of several techniques, in particular X-ray diffraction (XRD), Mössbauer spectroscopy and SQUID magnetization measurements. The XRD pattern of NZVI-containing filtration paper showed the characteristic peak at 52.5° (2*θ*, deg), signature that correspond to the (110) diffraction of body-centered cubic (bcc) of *α*-Fe phase (Figure 3).

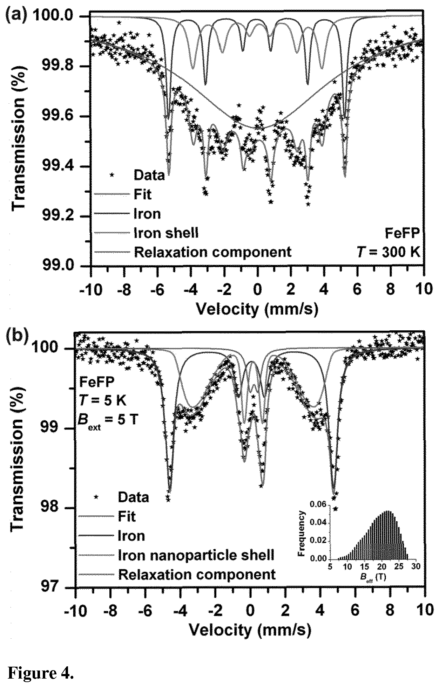

The (110) diffraction peak increased in intensity upon increasing the amount of the iron salt used within the initial FP immersion cycle and following NaBH₄ reduction. Therefore, through such simple synthetic procedure, it was possible to derive from the bottom-up approach the effective amount of NZVI loaded into FP (from 2.5% to 5% in weight for our tested composites). Diffraction signal due to other iron oxide phases (e.g., γ-Fe₂O₃/Fe₃O₄, α-Fe₂O₃) were absent in all tested preparations. These findings suggested that the NZVI nanoparticles intimately embedded into FP were less prone to undergo fast surface-passivation at ambient air, due to the emergence of some interactions between surface exposed iron nanoparticles and cellulose fibers. In this context, low temperature in-field ⁵⁷Fe Mössbauer spectroscopy was used to unveiling such interactions and to gain better knowledge of the electronic signature of NZVI sequestered into FP. The recorded Mössbauer spectrum at *T* = 5 K under 5 T external field (*B*ₑₓₜ) is shown in Figure 4 together with the fitting-deconvolution results (red-line). Three components contributed to the overall envelope, two sextets and one doublet, signatures that were found to hold for all preparations, irrespective of the loaded amount of NZVI into FP (from 2.5% to 5.0% NZVI in weight). One sextet encoded a high effective hyperfine magnetic field, *B*_{eff}*,* of 28.5 T and featured the typical signature of zero-valent-iron (Fe⁰) cores (Figure 4, blue line), with an isomer shift (*δ*) of 0.11 mm/s, and zero quadrupole splitting (Δ*E_{Q}*) value. The second magnetically split component reflected a fraction of Fe⁰ interacting with the sugar support (Figure 4, magenta-line) with *B*_{eff}, of 19.7 T, *δ* of 0.22 mm/s, and zero quadrupole splitting Δ*E_{Q}.* From these results, the NZVI-containing filtration paper can be described as composed by nanoparticles with a pseudo core-shell configuration, having the core formed by purely zero-valent iron and the shell of zero-valent iron in nature but with altered electronic features as also derived from the room-temperature Mössbauer spectrum.

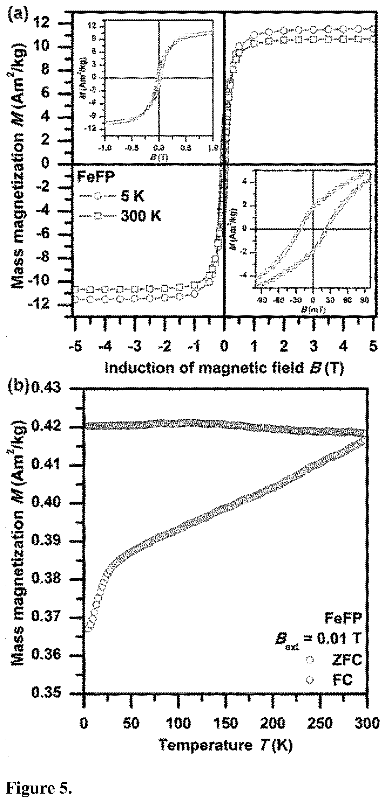

The witnessed effect is thought to originate from the intimate contacts of the surface exposed Fe⁰ metal with the sugar fibers, as shown in the representative TEM micrograph in Figure 2. The last spectral component observed, the doublet (Figure 4, green-line), reconcile with the fraction of NZVI characterised by small sizes (∼ 10 nm), which produced the fast relaxation of their NZVI shells in the Mössbauer time-scale (10⁻⁸ s). The Mössbauer parameters for such component were found to fall at 0.23 mm/s for *δ* and 0.45 mm/s for Δ*E_{Q}.* In this scenario, the external magnetic field (5 T) was still not strong enough to hinder superparamagnetic fluctuations of the shell spins. The dual nature of the electronic behaviour associated to the Fe⁰ in the NZVI-core and surface-exposed Fe⁰ located on the NZVI-shell was also confirmed in the composite's magnetic response by bulk susceptibility measurements. The magnetic behaviour of the NZVI-containing filtration paper with high loading (5.0 % of NZVI loaded, % in weight) showed symmetric hysteretic behaviour and saturation of magnetization at low field (< 1 T), reaching the value of 10.7 Am²/kg at room temperature that slightly increased to 11.5 Am²/kg at *T* = 5 K. The observed coercivity (*B*c) was found much higher than that reported for bulk iron (< 0.1 mT), namely 42 mT and 52 mT at *T* = 300 K and 5 K, respectively, implying magnetic hardening of the nanoparticle system. In particular, as shown in Figure 5, the zero-field-cooled (ZFC) magnetization curve measured on warming under an applied field of 0.01 T, showed the presence of a maximum at very low temperatures (∼30 K) corresponding to the blocking temperature of the NZVI nanoparticle shells. The magnetic core, on the contrary, was found in a magnetically blocked state already at *T* = 300 K, as witnessed from the absence of other maxima in the ZFC magnetization curve. These findings gave further support to the Mössbauer results and to the picture earlier given for NZVI as pseudo-core shell system.

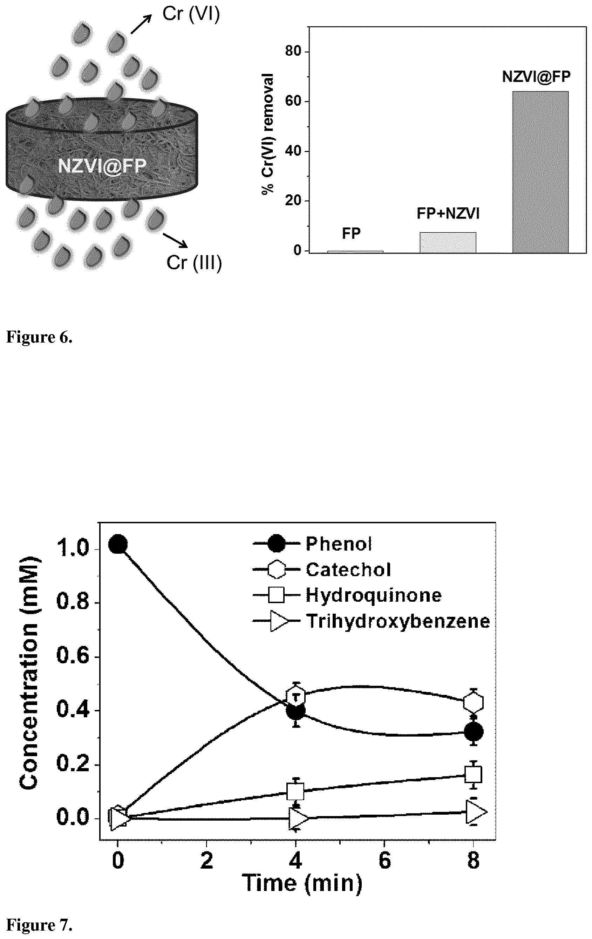

The specific architecture of composite obtained from the *in-situ* synthesis translated into the superior redox activity and proclivity to catalysis of this material with respect to bare NZVI in the environmental remediation processes of pollutants from water solutions. Noteworthy, it also prevented the severe release into the environment of NZVI from FP, whilst maintaining effective transport properties with high flux of the fluid systems, due to the porous nature of FP. The effectiveness of NZVI-containing filtration paper was tested in three scenarios, (i) reduction of the toxic hexavalent chromium into the environmentally friendlier Cr(III), (ii) proclivity of NZVI-containing filtration paper to activate hydroxylation reactions, in particular the *in-situ* conversion of phenols to catechols, and (iii) removal of As(V) by its reduction and sorption on NZVI-containing filtration paper. The results obtained for the Cr(VI) removal by simple filtration of gravity-feed solutions over the NZVI-containing filtration paper are shown in Figure 6. A freshly prepared stock solution of Cr(VI) (6.5 mg/L, 30 mL) was slowly poured through NZVI-containing filtration paper (∼5 min) and the filtrate was collected and characterized by UV-Vis absorption spectroscopy to determine the residual amount of Cr(VI). The observed efficiency in the Cr(VI) reduction by NZVI-containing filtration paper (expressed in %) was compared with the performance obtained for the composites assembled through an *ex-situ* approach (NZVI+FP) as well as bare FP. The NZVI+FP material was generated by simple impregnation of FP with separately and freshly synthesized NZVI nanoparticles (prepared by the following NaBH₄ reduction method: 1 g of FeCl₃·6H₂O was dissolved in 50 mL of ethanol; the reducing agent was prepared by dissolving 1 g of NaBH₄ in 50 mL of deionized water and added drop-wise to the Fe³⁺ solution; the mixture was allowed to settle for 15 min and the particles recovered via magnet were washed three times with absolute ethanol and then dried under vacuum; the FP was soaked in 40 mg of NZVI powder in 10 mL of EtOH and the material was sonicated for 15 min, followed by washing with ethanol and drying under vacuum; the amount of NZVI present in the FP was found to be ∼8 mg, equal to 3% of the total weight of the composite). It is important to underline that the final amount of loaded NZVI was found consistently lower in the case of the *ex-situ* synthesis (NZVI+FP), about 3 wt% on average, in comparison to the highest NZVI entrapment on FP obtained from the *in-situ* assembly (5 wt%). Looking at the process of Cr(VI) removal, the use of the NZVI-containing filtration paper allowed to reach the remarkable value of 64% after just three filtration cycles (Figure 6). On the contrary, the *ex-situ* material (NZVI+FP) showed, in such set-up, a decreased ability to reduce Cr(VI), and only 7.5% of hexavalent chromium could be successfully removed. Finally, the use of bare FP had, as expected, no visible effect on the reduction process of Cr(VI). While the different loading of NZVI into FP, from the *in-situ* to the *ex-situ* materials, may justify slight variations in the total amount of Cr(VI) removal by NZVI-modified filter paper (NZVI@FP), the witnessed differences are striking and suggest that the *in-situ* assembly process clearly generated a much more performant composite.

The ability of NZVI-containing filtration paper to act as catalyst in hydroxylation reactions was tested using the simple phenol as benchmark, which is a highly toxic organic pollutant with suspected mutagenic and carcinogenic properties, and by employing hydrogen peroxide as green-oxidant. The reaction conditions in those materials required the use of high temperatures, low pH and prolonged times. We found that NZVI-containing filtration paper was capable to promote conversion of phenol into less toxic catechols already at low temperature (40 °C), at neutral pH and after short time via simple filtration steps. Figure 7 summarizes the experimental findings with NZVI-containing filtration paper (5 wt % of NZVI) used as a membrane catalyst. Nearly 70 % of the initial amount of phenol was converted in 8 min, giving rise to catechol (47 %), hydroquinone (18 %) and trihydroxybenzene (3 %) after analyses by GC-MS. Catechol was the predominant product, thus the hydroxylation occurred preferentially in the *ortho*-position. Control experiments using bare FP (and hydrogen peroxide) showed, as expected, no ability to perform hydroxylation of phenol.

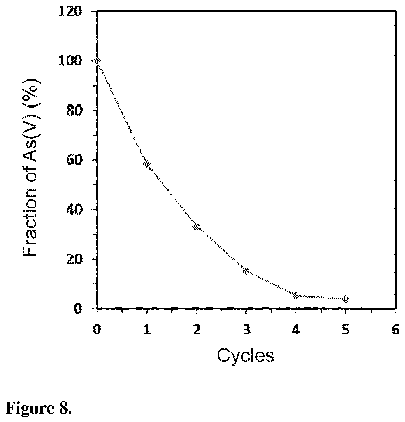

The ability of NZVI-containing filtration paper for removal of As(V) is depicted on Figure 8. A freshly prepared stock solution of As(V) (1 mg/L, 200 mL) was slowly poured through NZVI-containing filtration paper (5 cycles) and the filtrate was collected after each step and characterized by AAS to determine the residual amount of As(V). The nearly complete removal of arsenic was observed after fourth cycle.

### Example 2

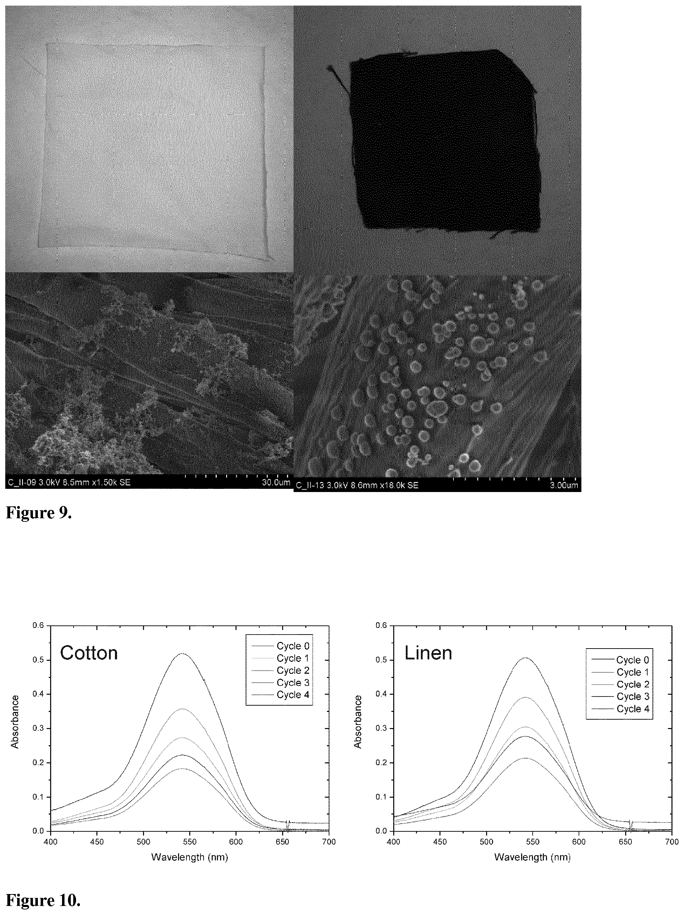

The preparation of NZVI embedded in cotton-fiber cloth according to the two-step procedure described in Example 1. The macroscopic optical changes of the cotton cloth are shown in Figure 9 (top) and the detailed scanning-electron microscopy (SEM) characterization is presented on Figure 9 (bottom) showing NZVI particles with a mean size of 300 nm. A freshly prepared stock solution of Cr(VI) (6.5 mg/L, 30 mL) was slowly poured through NZVI-containing cotton cloth and the filtrate was collected and characterized by UV-Vis absorption spectroscopy to determine the residual amount of Cr(VI). The observed efficiency in the Cr(VI) reduction by NZVI-containing cotton cloth is illustrated on Figure 10 (left).

### Example 3

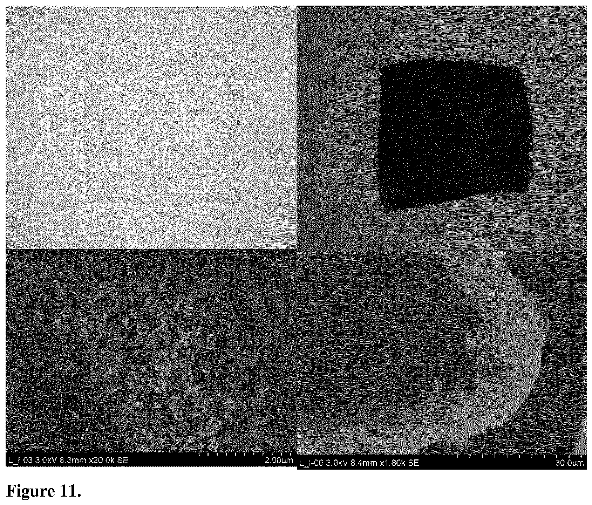

The preparation of NZVI embedded in linen-fiber cloth according to the two-step procedure described in Example 1. The macroscopic optical changes of the linen cloth are shown in Figure 11 (top) and the detailed SEM characterization is presented on Figure 11 (bottom) showing NZVI particles with a mean size of 250 nm. A freshly prepared stock solution of Cr(VI) (6.5 mg/L, 30 mL) was slowly poured through NZVI-containing linen cloth and the filtrate was collected and characterized by UV-Vis absorption spectroscopy to determine the residual amount of Cr(VI). The observed efficiency in the Cr(VI) reduction by NZVI-containing linen cloth is illustrated on Figure 10 (right).

## Claims

1. A composite material containing zerovalent iron particles having the dimensions of 5 to 500 nm, **characterized in that** it consists of a cellulose fibrous host material selected from a cotton-fiber cloth, a linen-fiber cloth, and a filter paper, and zerovalent iron particles having the dimensions of 5 to 500 nm, said particles consisting of iron.

2. A method of preparation of the material containing zerovalent iron particles having the dimensions of 5 to 500 nm of claim 1, **characterized in that** a cellulose fibrous material selected from a cotton-fiber cloth, a linen-fiber cloth, and a filter paper, is impregnated with an aqueous or Cl-C4-alcoholic iron salt solution, and subsequently the impregnated fibrous material is subjected to reduction by a reduction agent.

3. The method according to claim 2, wherein the iron salt is ferric halogenide.

4. The method according to any one of claims 2 or 3, wherein the reduction is carried out using sodium borohydride.

5. Use of the composite material according to claim 1 as a filter for wastewater treatment, in particular for remediation of heavy metals or oxoanions.

6. Use of the composite material according to claim 1 as a catalyst, in particular as a catalyst in hydroxylation reactions.

## Patentansprüche

1. Kompositmaterial, das Nullwertiges-Eisen-Partikel mit den Abmessungen von 5 bis 500 nm enthält und **dadurch gekennzeichnet ist, dass** es aus einem zellulosefaserigen Wirtsmaterial, das aus einem Baumwollfasertuch, einem Leinenfasertuch und einem Filterpapier ausgewählt ist, und Nullwertiges-Eisen-Partikel mit den Abmessungen 5 bis 500 nm besteht, wobei die Partikel aus Eisen bestehen.

2. Verfahren zur Herstellung des Materials nach Anspruch 1, das Nullwertiges-Eisen-Partikel mit den Abmessungen von 5 bis 500 nm enthält, **dadurch gekennzeichnet, dass** ein Cellulosefasermaterial, ausgewählt aus einem Baumwollfasertuch, einem Leinenfasertuch und einem Filterpapier, wird mit einer wässrigen oder C1-C4-alkoholischen Eisensalzlösung imprägniert, und anschließend wird das imprägnierte Fasermaterial einer Reduktion durch ein Reduktionsmittel unterzogen.

3. Verfahren nach Anspruch 2, wobei das Eisensalz Eisenhalogenid ist.

4. Verfahren nach einem der Ansprüche 2 oder 3, wobei die Reduktion unter Verwendung von Natriumborhydrid durchgeführt wird.

5. Verwendung des Kompositmaterials nach Anspruch 1 als Filter zur Abwasserbehandlung, insbesondere zur Sanierung von Schwermetallen oder Oxoanionen.

6. Verwendung des Kompositmaterials nach Anspruch 1 als Katalysator, insbesondere als Katalysator bei Hydroxylierungsreaktionen.

## Revendications

1. Matériau composite, contenant des particules de fer zérovalent ayant les dimensions de 5 à 500 nm, **caractérisé en ce qu'**il consiste en un matériau hôte fibreux cellulosique choisi parmi un tissu en fibre de coton, un tissu en fibre de lin et un papier filtre, et en des particules de fer zérovalent ayant les dimensions de 5 à 500 nm, lesdites particules étant constituées de fer.

2. Procédé de préparation du matériau contenant des particules de fer zérovalent ayant les dimensions de 5 à 500 nm selon la revendication 1, **caractérisé en ce qu'**un matériau fibreux cellulosique choisi parmi un tissu en fibre de coton, un tissu en fibre de lin et un papier filtre est imprégné d'une solution aqueuse ou alcoolique en C1-C4 du sel de fer, et ensuite le matériau fibreux imprégné est soumis à une réduction par un agent réducteur.

3. Procédé selon la revendication 2, dans lequel le sel de fer est un halogénure ferrique.

4. Procédé selon l'une quelconque des revendications 2 ou 3, dans lequel la réduction est effectuée à l'aide de borohydrure de sodium.

5. Utilisation du matériau composite selon la revendication 1 comme filtre pour le traitement des eaux usées, en particulier pour l'enlèvement de métaux lourds ou d'oxoanions.

6. Utilisation du matériau composite selon la revendication 1 comme catalyseur, de préférence comme catalyseur dans des réactions d'hydroxylation.
